# EUROPEAN PATENT APPLICATION

(11) **EP 4 785 872 A1**
(43) Date of publication of application: **05.08.2026**
(21) Application number: 25154867.3
(22) Date of filing: 30.01.2025
(51) Int. Cl.: A61B 8/00, A61B 8/08

(54) **SYSTEM AND METHOD OF ESTIMATING CALIPER PLACEMENT BASED ON USER PREFERENCES**

(71) Applicant: Koninklijke Philips N.V., 5656 AE Eindhoven (NL)
(72) Inventor: BAI, Xianghui, Eindhoven (NL); MENG, Yishuang, Eindhoven (NL); LIN, Qizhong, 5656AG Eindhoven (NL); SHIH, Cho-Chiang, Eindhoven (NL); CHEN, Jinning, Eindhoven (NL); XIE, Hua, Eindhoven (NL); ERRICO, Claudia, Eindhoven (NL); BRUNELLE, Elizabeth, Eindhoven (NL); JING, Xiang, Eindhoven (NL); DING, Jianmin, Eindhoven (NL)
(74) Representative: Philips Intellectual Property & Standards

(57) **Abstract**

A method for determining positions of calipers includes performing calibration to identify preferred placements of calipers in template ultrasound images. The method includes receiving ultrasound image of a patient, inputting the ultrasound image to a first machine learning model for automatically detecting an anatomical view of an anatomy and cropping the ultrasound image to show an ROI, retrieving a matching template ultrasound image that matches the detected anatomical view and preferred placements of the calipers, encoding the preferred placements to provide position embedding, encoding the matching template ultrasound image to provide a first image embedding, encoding the cropped ultrasound image to provide a second image embedding, inputting the position embedding, the first image embedding, and the second image embedding to a trained second machine learning model for predicting positions of calipers in the cropped ultrasound image. The method includes displaying the predicted positions of the calipers, enabling placement of the calipers at the predicted positions.

## Description

### FIELD OF THE INVENTION

The invention relates to the field of ultrasound imaging, and more specifically to estimating caliper placement for ultrasound imaging based on user preferences.

### BACKGROUND OF THE INVENTION

Artificial intelligence (AI) technology has been progressively integrated into ultrasound imaging systems to automate workflow for improved user productivity and inter-user acquisition consistency. One application is directed to replacing tedious manual measurements of anatomy dimensions with machine learning enabled auto-measure tools. Examples of anatomies requiring particularly tedious manual measurements include abdominal organ measurements in gastrointestinal (e.g., liver length, gallbladder wall thickness) and genitourinary (e.g., renal length), fetal biometric parameter measurements in maternal-fetal medicine (e.g., abdominal circumference, head circumference, femur length), and two-dimensional (2D) echocardiographic measurements in cardiovascular (e.g., left ventricular internal end diastolic dimension, left ventricular internal end systolic dimension).

The essence of auto-measure tools in ultrasound imaging is to train a machine learning model based on measurement behaviors of human clinicians, and to automatically place calipers for organ and anatomy size measurements. "Calipers" in this context are for example computer generated graphical measurement tools that graphically overlay an ultrasound image display for indicating the boundaries of measurements to be performed, as is well known in the art. Observations show that clinicians have different caliper placement preferences during organ measurement, especially for those measurement parameters where unique caliper positions do not exist. For example, for liver craniocaudal axis measurement, some clinicians prefer to place the first caliper on the top-left point along the liver contours, while others choose to place caliper on the intersection point of two tangent lines of liver contours. Both preferences are clinically recommended practices and widely used.

Similar measurement style differences are widely reported among clinicians and even among clinical sites. For example, instead of the standard oblique length measurement (typically used by North American ultrasound users), craniocaudal length has been proposed as a better metric for right liver lobe because it has higher inter-observer correlation. For gallbladder wall measurements in the sagittal plane, different clinicians may place calipers along different sections of the wall of the gallbladder. Given that the purpose of machine learning auto-measure tools is to reduce user operations and improve examination efficiency, it is unrealistic to expect that a machine learning model trained according to one measurement style will provide caliper placement locations that are acceptable to clinicians who prefer another measurements style. Training multiple machine learning models for multiple measurement styles is a straightforward solution. However, to do so is inflexible, and very time-consuming and expensive. Accordingly, a process is needed for adjusting and aligning outputs of auto-measure tools with user preferences without having to retrain for each individual preference.

### SUMMARY OF THE INVENTION

The invention is defined by the independent claims. Advantageous embodiments are provided in the dependent claims.

According to a representative embodiment, a method is provided for determining positions of calipers in ultrasound images. The method includes performing calibration to identify preferred placements of calipers by a user in multiple template ultrasound images showing multiple anatomical views of anatomies, respectively, where each anatomical view of the multiple anatomical views corresponds to a type of measurement to be performed on an anatomy; storing the preferred placements of the calipers in association with the multiple template ultrasound images, respectively; receiving an ultrasound image of a subject acquired during an ultrasound examination; inputting the ultrasound image of the subject to a trained first machine learning model for automatically detecting an anatomical view of an anatomy of interest in the ultrasound image using object detection and cropping the ultrasound image to separately show a region of interest (ROI) that includes the detected anatomical view, where the detected anatomical view corresponds to a type of measurement to be performed on the anatomy of interest; retrieving a matching template ultrasound image of the multiple template ultrasound images that matches the detected anatomical view of the anatomy of interest, and the preferred placements of the calipers stored in association with the matching template ultrasound image; encoding the preferred placements of the calipers to provide a position embedding representing positions of the preferred placements; encoding the matching template ultrasound image to provide a first image embedding representing the matching template ultrasound image; encoding the cropped ultrasound image separately showing the ROI to provide a second image embedding representing the cropped ultrasound image; inputting the position embedding, the first image embedding, and the second image embedding to a trained second machine learning model for predicting positions of calipers in the cropped ultrasound image based on the preferred placements of the calipers in the matching template ultrasound image; and displaying the predicted positions of the calipers in at least one of the ultrasound image or the cropped ultrasound image, enabling the user to accept or adjust the predicted positions of the calipers in the ultrasound image of the subject for measuring the anatomy of interest using the detected anatomical view.

In some embodiments, performing the calibration to identify the preferred placements of the calipers, comprises displaying the plurality of template ultrasound images showing an anatomical view of the plurality of anatomical views; and for each displayed template ultrasound image, receiving input from the user indicating positions of calipers for performing a measurement of the anatomy in the anatomical view shown in the displayed template ultrasound image, and storing the positions of the calipers as the preferred placements for performing the measurement of the anatomy.

In some embodiments, performing the calibration to identify the preferred placements of the calipers, comprises displaying the plurality of template ultrasound images showing an anatomical view of the plurality of anatomical views; and for each displayed template ultrasound image, displaying a plurality of predetermined positions of calipers for performing a measurement of the anatomy in the anatomical view shown in the displayed template ultrasound image, receiving input from the user selecting one of the predetermined positions of the plurality of predetermined positions of the calipers for performing the measurement of the anatomy, and storing the selected one of the predetermined positions of the calipers as the preferred placements for performing the measurement of the anatomy.

The trained first machine learning model may preferably be a You Only Look Once (YOLO) object detection model or a Single-Shot Multi-box Detection (SSD) model. Additionally or alternatively, the trained second machine learning model may preferably be a YOLO object detection model or an SSD model.

In some embodiments, encoding the preferred placements of the calipers to provide the position embedding comprises inputting coordinates of the preferred placements of the calipers to a mathematical model, which encodes the coordinates into a position multidimensional vector representing positions of the preferred placements.

In such embodiments, encoding the matching template ultrasound image to provide the first image embedding preferably comprises inputting the matching template ultrasound image to a trained image encoding model, which encodes the matching template ultrasound image into a first image multidimensional vector representing at least first image semantic information of the matching template ultrasound image.

More preferably, encoding the cropped ultrasound image separately showing the ROI to provide the second image embedding may comprise inputting the cropped ultrasound image to the trained image encoding model, which encodes the cropped ultrasound image into a second image multidimensional vector representing at least second image semantic information of the cropped ultrasound image.

In some embodiments, the trained second machine learning model may be a transformer decoder model.

In some embodiments, predicting the positions of the calipers in the cropped ultrasound image comprises inputting the position embedding, the first image embedding, and the second image embedding, and using the position embedding and the first image embedding as prompts to guide the trained second machine learning model to predict the positions of the calipers in the cropped ultrasound image.

Preferably, in such embodiments, the trained second machine learning model applies self-attention and cross-attention to the position embedding, the first image embedding, and the second image embedding to determine relationships within and between the position embedding, the first image embedding, and the second image embedding for predicting the positions of the calipers in the cropped ultrasound image.

According to another representative embodiment, a system is provided for determining positions of calipers in ultrasound images, the system comprising a display, a processing unit coupled to the display, and a non-transitory memory storing instructions that, when executed by the processing unit, cause the processing unit to perform the steps of a method of any of the above embodiments.

In some embodiments, the display is preferably adapted to display the plurality of template ultrasound images. More preferably, the display is further adapted to display, for each displayed template ultrasound image, a plurality of predetermined positions of calipers for performing a measurement of the anatomy in the detected anatomical view shown in the displayed template ultrasound image.

According to another representative embodiment, a system is provided for determining positions of calipers in ultrasound images, the system including a display, a processing unit coupled to the display, and a non-transitory memory. The non-transitory memory stores instructions that, when executed by the processing unit, cause the processing unit to perform calibration to identify preferred placements of calipers by a user in multiple template ultrasound images showing multiple anatomical views of anatomies, respectively, by displaying the multiple template ultrasound images on the display and receiving the preferred placements of the calipers in each anatomical view, where each anatomical view corresponds to a type of measurement to be performed on an anatomy; store the preferred placements of the calipers in association with the multiple template ultrasound images, respectively; receive an ultrasound image of a subject acquired during an ultrasound examination; input the ultrasound image of the subject to a trained first machine learning model for automatically detecting an anatomical view of an anatomy of interest in the ultrasound image using object detection and cropping the ultrasound image to separately show an ROI that includes the detected anatomical view, where the detected anatomical view corresponds to a type of measurement to be performed on the anatomy of interest; retrieve a matching template ultrasound image of the multiple template ultrasound images that matches the detected anatomical view of the anatomy of interest, and the preferred placements of the calipers stored in association with the matching template ultrasound image; encode the preferred placements of the calipers to provide a position embedding representing positions of the preferred placements; encode the matching template ultrasound image to provide a first image embedding representing the matching template ultrasound image; encode the cropped ultrasound image separately showing the ROI to provide a second image embedding representing the cropped ultrasound image; input the position embedding, the first image embedding, and the second image embedding to a trained second machine learning model for predicting positions of calipers in the cropped ultrasound image based on the preferred placements of the calipers in the matching template ultrasound image; and display the predicted positions of the calipers in at least one of the ultrasound image or the cropped ultrasound image on the display, enabling the user to accept or adjust the predicted positions of the calipers in the ultrasound image of the patient for measuring the anatomy of interest using the detected anatomical view.

In some embodiments, the instructions cause the processing unit to perform the calibration by receiving input from the user for each displayed template ultrasound image indicating positions of calipers on the display for performing a measurement of the anatomy in the detected anatomical view shown in the displayed template ultrasound image, and storing the positions of the calipers as the preferred placements for performing the measurement of the anatomy.

In some embodiments, the instructions cause the processing unit to perform the calibration by displaying a plurality of predetermined positions of calipers for each displayed template ultrasound image for performing a measurement of the anatomy in the detected anatomical view shown in the displayed template ultrasound image, receiving input from the user selecting one of the predetermined positions of the plurality of predetermined positions of the calipers for performing the measurement of the anatomy, and storing the selected one of the predetermined positions of the calipers as the preferred placements for performing the measurement of the anatomy.

In some embodiments, each of the trained first machine learning model and the trained second machine learning model is a You Only Look Once (YOLO) object detection model or a Single-Shot Multi-box Detection (SSD) model.

In some embodiments, the instructions cause the processing unit to encode the preferred placements of the calipers to provide the position embedding by inputting coordinates of the preferred placements of the calipers to a mathematical model, which encodes the coordinates into a position multidimensional vector representing positions of the preferred placements.

In such embodiments, the instructions preferably cause the processing unit to encode the matching template ultrasound image to provide the first image embedding by inputting the matching template ultrasound image to a trained image encoding model, which encodes the matching template ultrasound image into a first image multidimensional vector representing at least first image semantic information of the matching template ultrasound image.

More preferably in such embodiments, the instructions cause the processing unit to encode the cropped ultrasound image separately showing the ROI to provide the second image embedding by inputting the cropped ultrasound image to the trained image encoding model, which encodes the cropped ultrasound image into a second image multidimensional vector representing at least second image semantic information of the cropped ultrasound image.

According to another representative embodiment, a non-transitory computer readable medium storing instructions for determining positions of calipers in ultrasound images that, when executed by a processing unit, cause the processing unit to perform the steps of a method of any of the above embodiments.

According to another representative embodiment, a non-transitory computer readable medium is provided that stores instructions for determining positions of calipers in ultrasound images. When executed by a processing unit, the instructions cause the processing unit to perform calibration to identify preferred placements of calipers by a user in multiple template ultrasound images showing multiple anatomical views of anatomies, respectively, where each anatomical view corresponds to a type of measurement to be performed on an anatomy; store the preferred placements of the calipers in association with the multiple template ultrasound images, respectively; receive an ultrasound image of a subject acquired during an ultrasound examination; input the ultrasound image of the subject to a trained first machine learning model for automatically detecting an anatomical view of an anatomy of interest in the ultrasound image using object detection and cropping the ultrasound image to separately show an ROI that includes the detected anatomical view, where the detected anatomical view corresponds to a type of measurement to be performed on the anatomy of interest; retrieve a matching template ultrasound image of the multiple template ultrasound images that matches the detected anatomical view of the anatomy of interest, and the preferred placements of the calipers stored in association with the matching template ultrasound image; encode the preferred placements of the calipers to provide a position embedding representing positions of the preferred placements; encode the matching template ultrasound image to provide a first image embedding representing the matching template ultrasound image; encode the cropped ultrasound image separately showing the ROI to provide a second image embedding representing the cropped ultrasound image; input the position embedding, the first image embedding, and the second image embedding to a trained second machine learning model for predicting positions of calipers in the cropped ultrasound image based on the preferred placements of the calipers in the matching template ultrasound image; and display the predicted positions of the calipers in at least one of the ultrasound image or the cropped ultrasound image on the display, enabling the user to accept or adjust the predicted positions of the calipers in the ultrasound image of the patient for measuring the anatomy of interest using the detected anatomical view.

In some embodiments, the instructions cause the processing unit to encode the preferred placements of the calipers to provide the position embedding by inputting coordinates of the preferred placements of the calipers to a mathematical model, which encodes the coordinates into a position multidimensional vector representing positions of the preferred placements.

In some embodiments, the instructions cause the processing unit to encode the matching template ultrasound image to provide the first image embedding by inputting the matching template ultrasound image to a trained image encoding model, which encodes the matching template ultrasound image into a first image multidimensional vector representing at least first image semantic information of the matching template ultrasound image, and encode the cropped ultrasound image separately showing the ROI to provide the second image embedding by inputting the cropped ultrasound image to the trained image encoding model, which encodes the cropped ultrasound image into a second image multidimensional vector representing at least second image semantic information of the cropped ultrasound image.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a simplified block diagram of a system for determining positions of calipers in ultrasound images customized to user preferences determining positions of calipers in ultrasound images customized to user preferences, according to a representative embodiment.
Fig. 2A shows illustrative ultrasound image with first preferred caliper placements according to user preferences for liver cranio-caudal axis measurements.
Fig. 2B shows illustrative ultrasound image with second preferred caliper placements according to user preferences for liver cranio-caudal axis measurements.
Fig. 3 is a flow diagram of a method for identifying and cropping an anatomical view of an anatomy in an ultrasound image using a trained first machine learning model, according to a representative embodiment.
Fig. 4 is a flow diagram of a method for performing image encoding of ultrasound images, including a matching template ultrasound image and a cropped ultrasound image, to provide image embeddings, according to a representative embodiment.
Fig. 5 is a flow diagram of a method for predicting preferred placements of calipers in an ultrasound image showing an anatomical view of an anatomy of interest using a trained second machine learning model, according to a representative embodiment.
Fig. 6 is a flow diagram of a method of determining positions of calipers in ultrasound images customized to user preferences, according to a representative embodiment.
Fig. 7 shows a display displaying an illustrative original ultrasound image with predicted positions of the calipers determined for liver craniocaudal axis measurement, according to a representative embodiment.

### DETAILED DESCRIPTION OF EMBODIMENTS

Aspects of the disclosure may be supported by various information technology (IT) backends, including either or both local architectures, either as monoliths, networked, or a combination thereof, and hosted architectures, such as a software as a service (SaaS), platform as a service (PaaS), and/or infrastructure as a service (IaaS), or the like. In an example, a supporting infrastructure includes multiple interconnected layers respectively hosting, as an abstraction, various IT processes, services, accounts, and other management components.

Any of the steps described in relation to examples and/or training described below can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. As an example, a computer system performs logical processing based on digital signals received via an analogue-to-digital converter.

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide an artificial intelligence (AI) based method and system for automatically adjusting caliper positions in ultrasound images according to user preference without retraining the machine learning model. The AI enabled ultrasound auto-measure features are designed to predict the caliper positions used to measure the size of an organ. Clinicians have different caliper placement preferences during organ measurement. To train multiple machine learning models to support different caliper placement styles is time consuming, expensive and inflexible. The described embodiments herein employ an initial calibration step to collect user preferences, which are subsequently used as prompts to adjust auto-measure machine learning models to generate caliper positions aligned with user preferences.

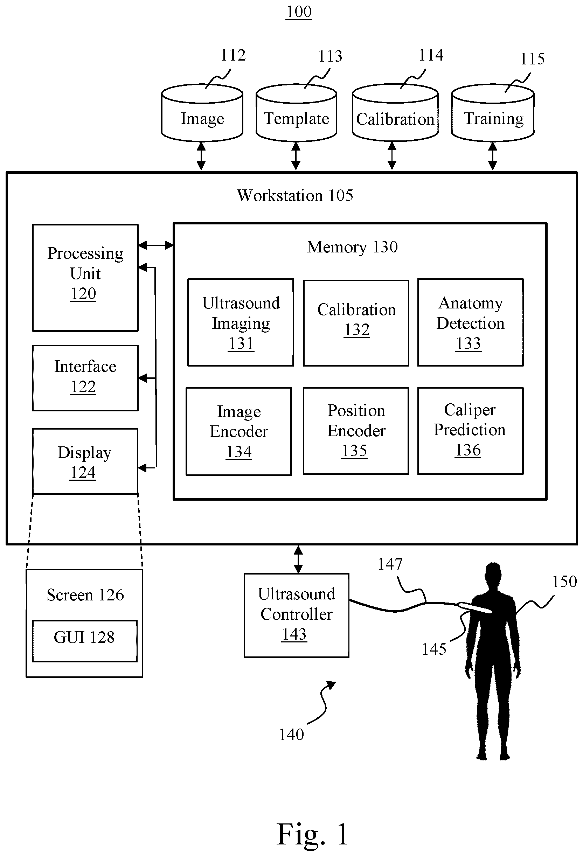

Fig. 1 is a simplified block diagram of a system for determining positions of calipers in ultrasound images customized to user preferences determining positions of calipers in ultrasound images customized to user preferences, according to a representative embodiment.

Referring to Fig. 1, system 100 includes a workstation 105 for implementing and/or managing the processes described herein with regard to estimating caliper placement in ultrasound images from an ultrasound imaging system 140 based on user preferences. The workstation 105 includes one or more processors indicated by processing unit 120, one or more memories indicated by memory 130, a user interface 122 and a display 124. The processing unit 120 communicates with the ultrasound imaging system 140 through an imaging interface (not shown). The ultrasound imaging system 140 includes an ultrasound controller 143 and an ultrasound probe 145 operable by an operator to obtain ultrasound images of various anatomies, including various organs, of a subject (patient) 150. The ultrasound probe 145 may be manipulated manually by the ultrasound user, automatically by a robot under control of a robot controller (not shown), or a combination of both.

The ultrasound probe 145 may include a 1D/2D matrix array of transducer elements, capable of scanning in two or three dimensions, for example, for emitting ultrasound waves into the body of the subject 150 and receiving echo signals in response. The transducer elements may include capacitive micromachined ultrasonic transducers (CMUTs) or piezoelectric transducers formed of materials such as lead zirconate titanate (PZT) or polyvinylidene difluoride (PVDF), for example, although other types of transducer material may be incorporated without departing from the scope of the present teachings. The transducer array may be coupled to a microbeamformer in the ultrasound probe 145, which controls transmission and reception of signals by the transducer elements.

The ultrasound probe 145 is connected to the ultrasound controller 143 via a probe cable 147. The ultrasound controller 143 is configured to control the ultrasound imaging process, and includes known elements for performing ultrasound imaging, such as a transmit/receive (T/R) switch configured to switch between transmission and reception modes, and a main beamformer configured to provide final beamforming. One of the functions performed by the ultrasound controller 143 is the direction in which beams are steered and focused. For example, beams may be steered straight ahead from (orthogonal to) the transducer array of the ultrasound probe 145, or at different angles for a wider field of view. Generally, the transmitting of ultrasound signals and the receiving and processing of echo signals in response is known, and therefore additional detail in this regard is not included herein. In various embodiments, all or part of the functionality of the ultrasound controller 143 may be implemented by the processing unit 120.

The memory 130 stores instructions executable by the processing unit 120. When executed, the instructions cause the processing unit 120 to implement one or more processes for estimating caliper placement in ultrasound images acquired by the ultrasound imaging system 140 based on user preferences established during initial calibration. The ultrasound images may be provided from the ultrasound imaging system 140 in real-time or near real-time during the scanning procedure, or may be retrieved from storage following the scanning procedure. For purposes of illustration, the memory 130 is shown to include software modules, each of which includes the instructions, executable by the processing unit 120, corresponding to an associated capability of the system 100.

The processing unit 120 is representative of one or more processing devices, and may be implemented by a general purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hardwired logic circuits, or combinations thereof. Any processor or processing unit herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 130 may include main memory and/or static memory, where such memories may communicate with each other and the processing unit 120 via one or more buses. The memory 130 may be implemented by any number, type and combination of random access memory (RAM) and read-only memory (ROM), for example, and may store various types of information, such as software algorithms, AI machine learning models, and computer programs, all of which are executable by the processing unit 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

The system 100 may also include databases for storing information that may be used by the various software modules of the memory 130. Representative databases may include an image database 112 for storing images from current and previous examinations of the subject 150, a template database 113 for storing ultrasound image templates for making various types of measurements in various anatomies, a calibration database 114 for storing ultrasound image templates including calibrated caliper placements for the various measurements, and a training database 115 for storing ultrasound images of anatomies for training machine learning models, as discussed below. For example, the image database 112 may include image data from previously obtained ultrasound images of the subject 150 and other situated subjects. Each of the image database 112, the template database 113, the calibration database 114, and the training database 115 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The databases comprise tangible storage mediums for storing data and executable software instructions and is non-transitory during the time data and software instructions are stored therein. The databases may be secure and/or encrypted, or unsecure and/or unencrypted. For purposes of illustration, the image database 112, the template database 113, the calibration database 114, and the training database 115 are shown as separate storage media, although it is understood that all or some of the databases may be combined with one another and/or included in the memory 130, without departing from the scope of the present teachings.

The processing unit 120 may include or have access to an AI engine, which may be implemented as software that provides artificial intelligence (e.g., deep learning, neutral network models) and applies machine learning described herein. The AI engine may reside in any of various components in addition to or other than the processing unit 120, such as the memory 130, an external server, and/or the cloud, for example. When the AI engine is implemented in a cloud, such as at a data center, for example, the AI engine may be connected to the processing unit 120 via the internet or other communication network using one or more wired and/or wireless connection(s). In various embodiments, all or part of the processes provided by first and second machine learning models, discussed below, may be implemented by the AI engine, for example. The training and execution of the first, second and image encoding machine learning models cannot practically be performed in the human mind.

The user interface 122 is configured to provide information and data output by the processing unit 120, the memory 130 and/or the ultrasound imaging system 140 to the user and/or for receiving information and data input by the user. That is, the user interface 122 enables the user to enter data and to control or manipulate aspects of the processes described herein, and also enables the processing unit 120 to indicate the effects of the user's input, which may include control or manipulation of the ultrasound probe 145 by the user. All or a portion of the user interface 122 may be implemented by a graphical user interface (GUI), such as GUI 128 viewable on the display 124. The user interface 122 may include one or more interface devices, such as a mouse, a keyboard, a trackball, a joystick, a microphone, a video camera, a touchpad, a touchscreen, voice or gesture recognition captured by a microphone or video camera, for example.

The display 124 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 124 includes a screen 126 for viewing ultrasound images of the subject 150, along with various features described herein to communicate to the user the degree of image degradation, if any, as well as the GUI 128 to enable the user to interact with the displayed images and features. In an embodiment, the ultrasound imaging system 140 may include a separate dedicated display for acquiring the ultrasound images, where dedicated display is also represented by the display 124.

Referring to the memory 130, the various modules store sets of data and instructions executable by the processing unit 120 to estimate caliper placement in ultrasound images based on user preferences established during initial calibration. The memory 130 includes ultrasound imaging module 131, which is configured to receive and process ultrasound images acquired by the ultrasound probe 145 of the ultrasound imaging system 140 to provide corresponding ultrasound image data. The ultrasound images are obtained by the user during corresponding ultrasound examinations, e.g., of the subject 150. The ultrasound images may be received in real-time or near real-time from the ultrasound imaging system 140, e.g., during a contemporaneous imaging session of the subject 150. The ultrasound images are displayed on the display 124, enabling the user to visualize the anatomy of the subject 150 while performing sweeps using the ultrasound probe 145. Alternatively, or in addition, the ultrasound images may be previously acquired images, obtained during a previous imaging session of the subject 150 and retrieved from storage (e.g., database 112). The ultrasound imaging module 131 may also store data associated with the ultrasound images, such as time and date of image acquisition, identification of the ultrasound imaging system 140, and identification of the ultrasound operator who acquired the ultrasound images using the ultrasound imaging system 140.

Calibration module 132 provides instructions for collecting and/or storing user preferences of caliper placements in template ultrasound images for performing various measurements of anatomy shown in the template ultrasound images, respectively. That is, prior to ultrasound imaging of the subject 150, the calibration module 132 performs calibration by prompting the user to provide preferences with regard to positions of the calipers placed on the template ultrasound images, where the caliper placements represent the user's particular measurement style. The template ultrasound images respectively show anatomical views of anatomies, where each anatomical view corresponds to a type of measurement to be performed on the anatomy. Each anatomical view is a view from an angle that provides an imaging plane in which a corresponding measurement of the anatomy is to be performed. Therefore, when a particular anatomy requires multiple measurements from different views/angles during an ultrasound imaging study, the calibration requires the user to identify preferred caliper placement in multiple images of the anatomy corresponding to the different views/angles.

To perform calibration, unmarked template ultrasound images are retrieved from one or more previously populated template databases, indicated by representative template database 113. The ultrasound images in the template database 113 may be provided by previously collected standard anatomy measurement images from clinical practice, for example. In the template database 113, there is at least one template ultrasound image showing an anatomical view for each type of measurement of each anatomy that the user will potentially encounter, depending on the user's specialty. For large organizations, such as hospitals, clinics or large practices, the template database 113 may be populated with template ultrasound images from numerous medical specialties in order to accommodate a broad range of services. Examples of such medical specialties include general imaging (GI), genitourinary (GUT), cardiac, and maternal-fetal medicine, mentioned above.

During calibration, the instructions of the calibration module 132 cause the processing unit 120 to collect the preferred caliper placements from the user for each of the template ultrasound images. Initially, the user identifies at least one specialty for which the system 100 is to be calibrated, depending on the user's training and expertise. The processing unit 120 responds to this information by accessing the template database 113 and downloading a set of template ultrasound images of anatomies corresponding to each identified specialty. The processing unit 120 displays the template ultrasound images on the display 124, where each template ultrasound image provides an anatomical view for making a particular measurement of the anatomy, as discussed above. Depending on the number template ultrasound images needed to complete the calibration, the template ultrasound images may be displayed on the display 124 one at a time or simultaneously for interaction with the user.

In an embodiment, the user identifies positions (key points) in each template ultrasound image as preferred placements of calipers for performing the measurement of the anatomy shown in the template ultrasound image. The user may indicate the preferred placements of the calipers using the user interface 122 and/or the GUI 128. For example, the user may point and click a mouse at the positions on the display 124 where the calipers are to be placed in each template ultrasound image. The preferred placements of the calipers are stored in association with each of the template ultrasound images in a calibration database 114 for ultimately performing measurements of the anatomy. The calibration database 114 may be a relational database, for example. Also, in an embodiment, the calibration database 114 may be the same as the template database 113.

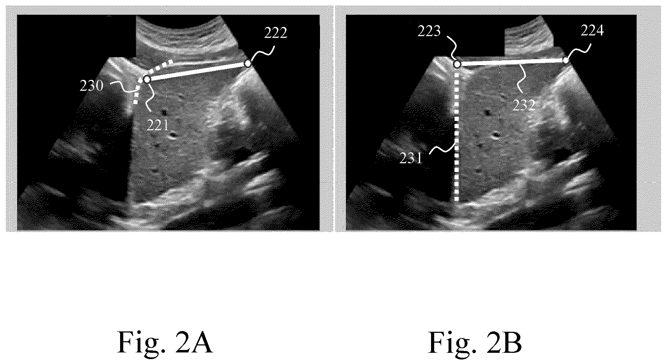

Figs. 2A and 2B show illustrative ultrasound images with different preferred caliper placements according to user preferences for liver cranio-caudal axis measurements. In particular, Fig. 2A depicts preferred placements of first and second calibers 221 and 222 at key points in an ultrasound image of a liver in which the first caliper 221 is placed on a top-left point at the center of arc 230 along the liver contours, indicated by a dashed line. In comparison, Fig. 2B depicts alternative preferred placements of third and fourth calibers 223 and 224 at key points in an ultrasound image of the liver in which the third caliper is placed on an intersection point of two tangent lines 231 and 232 of the liver contours. Both preferred placements follow clinically recommended practices, so selection of preferred placements comes down to user preference.

In another embodiment, each of the template ultrasound images is displayed together with multiple predetermined positions (key points) of calipers indicated in the anatomy for performing a corresponding measurement of the anatomy. The user then selects the preferred placements of the calipers from among the displayed predetermined positions using the user interface 122 and/or the GUI 128. For example, the user may point and click a mouse at each of the predetermined positions on the display 124 to be selected. Typically, a measurement requires at least two calipers, so the user may select at least two of the predetermined positions for making one measurement. Alternatively, predetermined positions of the calipers may be provided in sets, in which case the user may select one of the sets of predetermined positions. The preferred placements of the calipers are stored in association with each of the template ultrasound images, e.g., in the template database 113, for ultimately performing measurements of the anatomy.

Accordingly, as a result of calibration, the template database 113 stores template ultrasound images for performing various measurements in anatomical views of various anatomies, together with the user's individualized preferred caliper placements. For example, template ultrasound images corresponding to the GI specialty may show preferred caliper placements in anatomical views for measuring the liver (e.g., craniocaudal length and transverse diameter), the gallbladder (e.g., gallbladder wall thickness), and the urinary bladder (e.g., anteroposterior, transverse, and height). Template ultrasound images corresponding to the GUT specialty may show preferred caliper placements in anatomical views for measuring the kidneys (e.g., renal length), the spleen (e.g., length, width, height), and the prostate (e.g., length, width, height), for example. Template ultrasound images corresponding to the cardiac specialty may show preferred caliper placements in anatomical views for measuring the left and right ventricles (e.g., wall thickness, septal thickness) and the left and right atrium (e.g., diameter, minor axis dimension), for example. Template ultrasound images corresponding to the maternal-fetal medicine specialty may show preferred caliper placements in anatomical views for measuring the fetus at various stages (e.g., crown-rump length (CRL), biparietal diameter (BPD), head circumference (HC), femur length (FL) abdominal circumference (AC)), for example. Of course template ultrasound images may be provided for other types of measurements in these medical specialties, as well as for measurements in other medical specialties, without departing from the scope of the present teachings.

Following calibration, an ultrasound image acquired during an ultrasound examination of the subject 150 is received. The ultrasound image may be received during the ultrasound examination, e.g., from the ultrasound imaging system 140, or retrieved from memory, e.g., image database 112, following the ultrasound examination. The ultrasound image is input to anatomy detection module 133, which provides a first machine learning model for automatically detecting an anatomical view of an anatomy of interest in the ultrasound image and automatically cropping the input ultrasound image to separately show a region of interest (ROI), where the ROI is tailored to narrowly include the detected anatomical view without aspects of the ultrasound image showing other anatomies. In various embodiments, the first machine learning model may be a single, comprehensive model that detects all different anatomical views in multiple different anatomies. Alternatively, the first machine learning model may be a dedicated model that detects all anatomical view in one anatomy or just one anatomical view in one anatomy, in which case the first machine learning model does not need to first identify the anatomy and/or the particular anatomical view. The first machine learning model may detect the anatomical view using object detection, for example. As discussed above, the anatomical view is a view from an angle by the ultrasound probe 145 that provides an imaging plane in which a corresponding measurement of the anatomy of interest is to be performed.

Generally, the first machine learning model receives ultrasound image data of the ultrasound image as input, identifies the anatomical view in the anatomy of interest, crops the ultrasound image to the ROI to focus in on the anatomical view, and provides the cropped ultrasound image as output, as discussed below. The first machine learning model may be implemented as any suitable type of trainable machine learning model (e.g., deep learning model), such as a convolutional neural network (CNN), an artificial neural network (ANN), a recurrent neural network (RNN), a vision transformer, or a U-net model, for example. The first machine learning model may be a deep learning model, which refers to neural network with large numbers of layers and parameters that directly enable tasks such as classification and regression, for example, as would be apparent to one skilled in the art. In various embodiments, the trained first machine learning model may be a You Only Look Once (YOLO) object detection model or a Single-Shot Multi-box Detection (SSD) model, for example, although other types of object detection models may be implemented without departing from the scope of the present teachings.

The first machine learning model is previously trained in a supervised fashion using a first training data set including labelled ultrasound image data from, for example, thousands of training ultrasound images of different anatomical views in different anatomies, respectively. That is, each training ultrasound image is labeled to identify any anatomies shown in the image and the anatomical view of the anatomy from which the ultrasound image of the anatomy was acquired. Each anatomy includes an outer boundary, which is detectable by the first machine learning model to identify the anatomy. Alternatively, or in addition, the outer boundaries of the anatomy may also be labeled. When the first machine learning model is a dedicated model, directed to one or more anatomical views in one anatomy, the first training dataset includes only labelled ultrasound image data related to that anatomy and/or anatomical view. In any case, the labels may be added by experts in ultrasound imaging, such as radiologist and sonographers, for example, or may be labeled automatically through techniques such as ground truth automation, as would be apparent to one skilled in the art. The labeled first training data set may be referred to as ground truth data. The first training data set for training the first machine learning model may be stored in the training database 115, for example. In an embodiment, the first machine learning model may use bounding box regression loss and anatomy view classification loss (e.g., mean squared error loss, cross entropy loss) as the loss function, for example, during training.

The first machine learning model is thus trained end-to-end using the first training data set to detect and identify the anatomical views in the training ultrasound images, including detecting the outer boundaries of the corresponding anatomies, e.g., using object detection. At inference, after being trained on the first training data set, the first machine learning model is able to process new input ultrasound images and predict corresponding anatomical views based on the same. The first machine learning model also crops the new ultrasound images to separately provide the ROI specifically including the identified anatomical view of the anatomy. For example, the first machine learning model may use a bounding box to select the outer boundary of the identified anatomy to provide the ROI. The purpose of the detect and crop operation is to enable alignment of an input ultrasound image from an ultrasound examination with a calibrated template ultrasound image that matches the same anatomical view, discussed below, such that only the anatomical view of the anatomy of interest is included in both images, making for a more precise match.

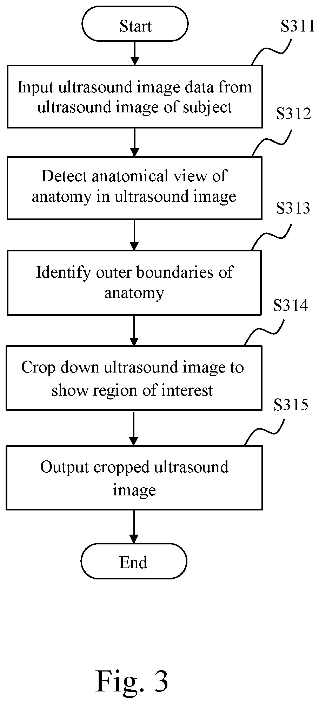

Fig. 3 is a flow diagram of a method for identifying and cropping an anatomical view of an anatomy in an ultrasound image using the trained first machine learning model provided by the anatomy detection module 133, according to a representative embodiment. That is, during inference, the trained first machine learning model receives as input the ultrasound image data from an ultrasound image of the subject 150 in block S311. In block S312, the trained first machine learning model searches the ultrasound image data and detects an anatomical view of an anatomy, and in block S313, identifies outer boundaries of the anatomy. In block S314, the trained first machine learning model crops the ultrasound image down to separately show an ROI that narrowly includes the identified anatomical view within the outer boundaries of the anatomy. That is, the trained first machine learning model removes portions of the ultrasound image outside the outer boundary of the anatomy to provide a cropped image separately showing the ROI. In block S315, the trained first machine learning model outputs the cropped ultrasound image.

Referring again to the calibration module 132, a matching template ultrasound image is identified from among the template ultrasound images stored in the template database 113 that matches the detected anatomical view of the anatomy shown in the cropped ultrasound image. The matching template ultrasound image may be matched based on input from the user at the user interface 122 and/or the GUI 128 expressly identifying the type of measurement to be performed using the ultrasound image provided to the trained first machine learning model. Alternatively, the matching template ultrasound image may be matched based on the anatomical view of the anatomy as detected by the trained first machine learning model itself. As mentioned above, the matching template ultrasound image substantially aligns with the cropped version of the input ultrasound image.

Image encoder module 134 is configured to provide a first image embedding representing the matching template ultrasound image and a second image embedding representing the cropped ultrasound image by respectively encoding features of the matching template ultrasound image identified by the calibration module 132 and features of the cropped ultrasound image separately showing the ROI output by the anatomy detection module 133. In an embodiment, the image encoder module 134 provides an image encoding model, which may be implemented as any suitable type of trainable machine learning model (e.g., deep learning model), such as a CNN, an ANN, an RNN, a vision transformer or auto-encoder, for example. The image encoding model is configured to encode pixels in the matching template ultrasound image into a first image multidimensional vector as the first image embedding, and to encode pixels in the cropped ultrasound image into a second image multidimensional vector as the second image embedding. Accordingly, the first image embedding is a numeric representation of the matching template ultrasound image, and the second image embedding is a numeric representation of the cropped ultrasound image, where the numeric representations encode the semantics of contents in these images, respectively. Each of the first and second image multidimensional vectors may include 128 dimensions, for example.

In an embodiment, the image encoding model may be a Vision Transformer (ViT) model, for example, as described by A. Dosovitskiy et al., "An Image is Worth 16x16 Words: Transformers for Image Recognition at Scale," International Conference on Learning Representations (ICLR) (2021), which is hereby incorporated by reference in its entirety. The ViT model effectively splits each of the matching template ultrasound image and the cropped ultrasound image into respective patches, encodes the patches into a sequence of linear embeddings of the patches (vectors) containing semantic information of the images, and provides the sequence of linear embeddings as an input to a Transformer model previously trained on a very large image encoding training data set. The image patches are treated by the Transformer model as tokens (words) in a natural language processing (NLP) application. Of course other types of image encoding models may be incorporated without departing from the scope of the present teachings, as would be apparent to one skilled in the art.

The image encoding model is previously trained on image encoding in a supervised fashion using training ultrasound images in a second training data set, which is very large (e.g., millions of images). The second training data set may be stored in the training database 115, for example. The image encoding model is part of a second machine learning model for predicting caliper placements, as discussed below, and therefore these models may be trained together. At inference, the image encoding model is able to input each of the matching template ultrasound image and the cropped ultrasound image, encode the matching template ultrasound image and the cropped ultrasound image as vectors, and output semantic information from the vectors as the first image embedding of the matching template ultrasound image and the second image embedding of the cropped ultrasound image, respectively, for example.

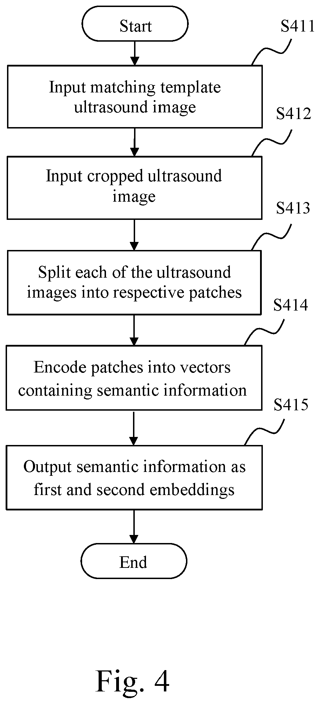

Fig. 4 is a flow diagram of a method for performing image encoding of ultrasound images, including the matching template ultrasound image and the cropped ultrasound image, to provide image embeddings, according to a representative embodiment. That is, during inference, the trained image encoding model receives as input the matching template ultrasound image from the calibration module 132 in block S411, and the cropped ultrasound image from the anatomy detection module 133 in block S412. In block S413, the trained image encoding model splits each of these ultrasound images into respective patches. In block S414, the trained image encoding model encodes the patches into vectors containing semantic information. In block S415, the semantic information is output as the first image embedding of the matching template ultrasound image and the second image embedding of the cropped ultrasound image, respectively.

Position encoder module 135 is configured to encode the preferred placements of the calipers to provide a position embedding that represents positions of the preferred placements. The preferred placements are the caliper positions previously stored in association with the matching template ultrasound image during calibration. The position encoder module 135 provides a position encoding model, which could be a mathematical model or a trainable model configured to encode coordinates (e.g., x, y coordinates) of the preferred placements of the calipers associated with the matching template ultrasound image into a position multidimensional vector as the position embedding associated with the matching template ultrasound image, as would be apparent to one skilled in the art. The position multidimensional vector may include the same number of dimensions as the first and second image multidimensional vectors, e.g., 128 dimensions, discussed above.

The position encoding model may be a Transformer model based on self-attention mechanisms, for example, as described by A. Vaswani et al., "Attention Is All You Need," 31st Conference on Neural Information Processing Systems (NIPS 2017), which is hereby incorporated by reference in its entirety. Positional encoding describes the location or position of an entity in a sequence so that each position is assigned a unique representation. Transformers use a positional encoding scheme, in which each position/index is mapped to a vector. Hence, the output of the positional encoding model is a matrix, where each row of the matrix represents an encoded object of the sequence summed with its positional information. Of course other types of position encoding models may be incorporated without departing from the scope of the present teachings, as would be apparent to one skilled in the art.

The positions of calipers in the cropped ultrasound image are predicted based on the preferred placements of the calipers in the matching template ultrasound image using the outputs of the image encoder module 134 and the position encoder module 135, which may be referred to as decoding. In particular, caliper prediction module 136 provides a second machine learning model configured to predict positions of calipers in the cropped ultrasound image based on the preferred placements of the calipers in the matching template ultrasound image, such that the placement of the calipers in the cropped ultrasound image are specific to the previously calibrated preferences of the user. Generally, the second machine learning model receives as input the position embedding, the first image embedding, and the second image embedding, and predicts the positions of the calipers in the cropped ultrasound image using the position embedding and the first image embedding as guidance or prompts to guide the trained second machine learning model. The trained second machine learning model may apply self-attention and cross-attention to the position embedding, the first image embedding, and the second image embedding to determine relationships within and between the position embedding, the first image embedding, and the second image embedding for predicting the positions of the calipers in the cropped ultrasound image. The second machine learning model may be implemented as any suitable type of trainable machine learning model (e.g., deep learning model), such as an RNN, a CNN, an ANN, or a vision transformer, for example. In various embodiments, the trained second machine learning model likewise may be a Transformer model, as discussed above with regard to the position encoding model.

The second machine learning model is previously trained in a supervised fashion, together with the image encoding model discussed above, using the second training data set including labelled ultrasound image data from thousands of training ultrasound images of calipers in different anatomies, respectively. The training ultrasound images are cropped to separately show ROIs corresponding to anatomical views of anatomies. That is, each training ultrasound image is labeled to identify the anatomy shown in the image, the anatomical view from which the ultrasound image of the anatomy was acquired, and different positions of calipers transferred from each template ultrasound image for measuring the anatomical view of the anatomy in the ultrasound image. For each combination of a training ultrasound image and preferred placements of calipers in a template ultrasound image (which could be more than one because of different preferences), a corresponding label indicating where to place calipers in the training ultrasound image is provided to supervise the second machine learning model to learn the relationship between the input training ultrasound image and the preferred placements of the calipers in the template ultrasound image, and then to use the preferred placements of the calipers to guide its prediction on a new input image at inference, discussed below. The labels may be added by experts in ultrasound imaging, such as radiologist and sonographers, for example, or may be labeled automatically through techniques such as ground truth automation, as would be apparent to one skilled in the art. The labeled second training data set may be referred to as ground truth data. The second training data set for training the second machine learning model may be stored in the training database 115, for example. In an embodiment, the second machine learning model may use key point regression loss (e.g., mean squared error loss) as the loss function, for example, during training.

The second machine learning model is thus trained end-to-end using the second training data set to estimate positions of calipers (key points) in ultrasound images of the subject 150. At inference, after being trained on the second training data set, the second machine learning model is able to process new cropped ultrasound images and new matching template ultrasound images corresponding to the cropped ultrasound images to predict preferred placements of the calipers from the matching template ultrasound images in the cropped ultrasound images, respectively.

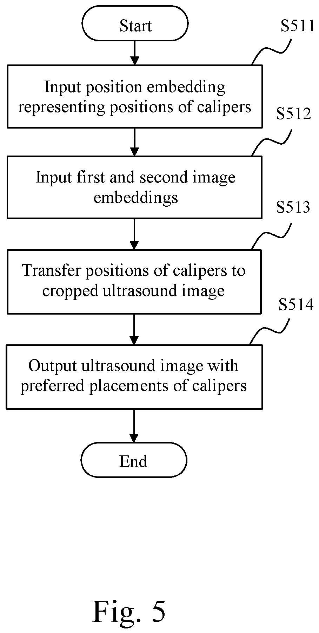

Fig. 5 is a flow diagram of a method for predicting preferred placements of calipers in an ultrasound image showing an anatomical view of an anatomy of interest using the trained second machine learning model provided by the caliper prediction module 136, according to a representative embodiment. That is, during inference, the trained second machine learning model receives as input the position embedding representing the positions of the calipers in the matched template ultrasound image from the position encoding model in block S511, and the first image embedding representing the matching template ultrasound image and the second image embedding representing the cropped ultrasound image from the encoding model block S512. In block S513, the trained second machine learning model transfers the positions of the calipers in the matching template ultrasound image to the cropped ultrasound image to show the user's preferred placement of the calipers in the cropped ultrasound image. In an embodiment, the second machine learning model may apply self-attention and cross-attention to the position embedding, the first image embedding, and the second image embedding to determine relationships within and between the position embedding, the first image embedding, and the second image embedding for predicting the preferred placements of the calipers in the cropped ultrasound image. That is, with regard to the first and second image embeddings of the input ultrasound image and the template ultrasound image (N-dimensional vectors in which each dimension represents some feature of the image), the second machine learning model uses attention to find or "pay attention" to dimensions that have strong relationships with final caliper position prediction. Self-attention applies inside each of the first and second image embeddings and cross-attention applies between the input ultrasound image and the template ultrasound image. In block S514, the trained second machine learning model outputs the cropped ultrasound image with the preferred placements of the calipers indicated as key points.

The predicted preferred placements of the calipers are displayed in the cropped ultrasound image and/or the original input ultrasound image (without the cropping) on the display 124. That is, the coordinates of predicted preferred placements of the calipers are based on the cropped ultrasound image, but they can be reprojected to corresponding coordinates of the input ultrasound image because the position of the ROI in the input ultrasound image is known. Displaying the predicted preferred placements of the calipers on the original input ultrasound image provides a larger field of view for the user. The user is then able to review the predicted preferred placements of the calipers, and if acceptable, to place the calipers in the ultrasound image used by the ultrasound imaging system 140 to enable the ultrasound imaging system 140 to measure the anatomy of interest. Alternatively, in response to the user accepting the predicted preferred placements of the calipers, the processing unit 120 may automatically transfer the positions of the calipers to the ultrasound imaging system 140 for making the measurement. Also, the user may make adjustments to the predicted preferred placements of the calipers, e.g., using the user interface 122 and/or the GUI 128, before placing the calipers in the ultrasound image for measuring the anatomy of interest.

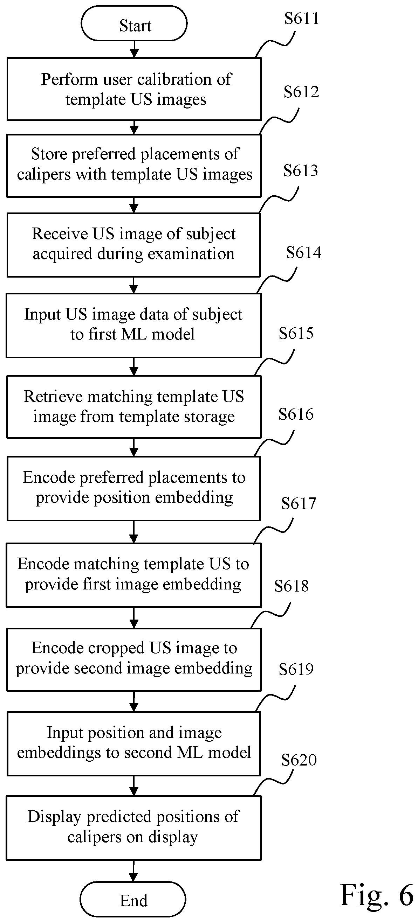

Fig. 6 is a flow diagram of an overall method of determining positions of calipers in ultrasound images customized to user preferences, according to a representative embodiment. The method may be implemented at least in part using instructions stored in memory 130 and executable by the processing unit 120 in the system 100, for example.

Referring to Fig. 6, the method for determining positions of calipers in ultrasound images includes initially performing user specific calibration in block S611. The calibration is performed by a user and identifies the user's preferred placements of calipers in multiple template ultrasound images showing multiple anatomical views of various anatomies, respectively. Each anatomical view corresponds to a type of measurement to be performed on the anatomy with which the anatomical view is associated. The user will perform a calibration for preferred placement of calipers in ultrasound images showing all types of anatomy the user expects to encounter, e.g., depending on the user's specialty, respectively. For example, for a user who practices renal medicine, the calibration may include the user identifying preferred placements of calipers in template ultrasound images showing appropriate anatomical views of kidney anatomy with calipers placed in preferred positions for making kidney length, width, and height measurements, where each of the anatomical views is a sagittal or transverse view of kidney. Additional template ultrasound images of GI anatomy may enable liver measurements, gallbladder measurements, spleen measurements, and aorta measurements, respectively, for example.

As discussed above, the calibration may be performed by displaying template ultrasound images showing anatomies to the user. For each displayed template ultrasound image, the user indicates positions of calipers for performing the measurement of the anatomy shown in the displayed template ultrasound image. The indicated positions of the calipers stored as the preferred placements for performing the measurement of the anatomy. Alternatively, the calibration may be performed by displaying the template ultrasound images showing anatomies to the user, where each displayed template ultrasound image includes multiple different predetermined positions of calipers for performing the measurement of the anatomy shown in the displayed template ultrasound image. In response to prompts, the user selects one or more of the predetermined positions of the calipers for performing the measurement of the anatomy. The indicated positions of the calipers stored as the preferred placements for performing the measurement of the anatomy.

In block S612, the preferred placements of the calipers are stored in association with the template ultrasound images, respectively. The stored calibrated template ultrasound images are retrievable based on the types of measurements.

In block S613, an ultrasound image of a subject (patient), acquired during an ultrasound examination, is received. The ultrasound image may be received from an ultrasound imaging system contemporaneously with the ultrasound examination, or may be retrieved from memory of images from previous ultrasound examinations.

In block S614, ultrasound image data from the ultrasound image of the subject are input to a trained first machine learning model. The trained first machine learning model is configured to automatically detect an anatomical view of an anatomy of interest in the ultrasound image data using object detection, and to automatically crop the ultrasound image to separately show a region of interest (ROI) that includes the detected anatomical view, as discussed above with reference to Fig. 3. The detected anatomical view corresponds to a type of measurement to be performed on the anatomy of interest. The trained first machine learning model outputs the identity of the detected anatomical view and the ROI of the anatomy showing the detected anatomical view.

In block S615, a matching template ultrasound image is retrieved from the template storage of template ultrasound images that matches the detected anatomical view of the anatomy of interest. The matching template ultrasound image shows the preferred placements (key points) of the calipers as stored in association with the matching template ultrasound image during the calibration discusses above with reference to block S611. In an embodiment, the user is queried regarding the type of measurement being performed, which necessarily includes identifying the anatomy and the anatomical view of the anatomy. The matching template ultrasound image is then matched and retrieved from the template storage in response to the user's input identifying the type of measurement. For example, a GUI may present multiple buttons or a drop-down list identifying types of measurements on a display, in which case the type of measurement and corresponding matching template ultrasound image are identified in response to the user selecting one of the buttons or entry in the drop-down list. Examples of the different types of measurements with corresponding template ultrasound images, which may be selectable as buttons or list entries, include craniocaudal length and transverse diameter measurements of the liver, wall thickness measurement of the gallbladder, anteroposterior, transverse and height measurements of the urinary bladder, renal length measurement of the kidney, and length, width and height measurements of the spleen, although other types of measurements may be incorporated without departing from the scope of the present teachings. In alternative embodiment, the matching template ultrasound image is matched and retrieved from the template storage automatically in response to the output of the trained first machine learning model identifying the detected anatomy of interest.

In block S616, the preferred placements of the calipers are encoded to provide a position embedding representing positions of the preferred placements in the matching template ultrasound image. In an embodiment, encoding the preferred placements of the calipers to provide the position embedding may include inputting coordinates of the preferred placements of the calipers to a mathematical model or a trainable model, which encodes the coordinates into a position multidimensional vector representing positions of the preferred placements.

In block S617, the matching template ultrasound image is encoded to provide a first image embedding representing the matching template ultrasound image, as discussed above with reference to Fig. 4. In an embodiment, encoding the matching template ultrasound image to provide the first image embedding may include inputting the matching template ultrasound image to a trained image encoding model, such as a CNN, an ANN, an RNN, a vision transformer or an auto-encoder, for example, as discussed above. The first image embedding refers to a numeric representation of the matching template ultrasound image. For example, the image encoding model encodes the matching template ultrasound image into a first image multidimensional vector representing at least first image semantic information of the matching template ultrasound image. Each dimension of the multidimensional vector may be represented by a predetermined number of bits (e.g., 8 bits, or 16 bits). More particularly, the image encoding model may be trained to extract task-dedicated semantic information, which in this case, is prediction of caliper placement, so the trained encoder will try to find features in the matching template ultrasound image related to caliper location.

In block S618, the cropped ultrasound image separately showing the ROI is encoded to provide a second image embedding representing the cropped ultrasound image, as discussed above with reference to Fig. 4. In an embodiment, encoding the cropped image to provide the second image embedding may include inputting the cropped ultrasound image to a trained image encoding model, such as a CNN, an ANN, an RNN, a vision transformer or an auto-encoder, for example, as discussed above. The trained image encoding model may be the same trained image encoding model used to provide the first image embedding, for example. The second image embedding likewise refers to a numeric representation of the cropped ultrasound image. For example, the image encoding model encodes the cropped ultrasound image into a second image multidimensional vector representing at least second image semantic information of the cropped ultrasound image. Again, each dimension of the multidimensional vector may be represented by a predetermined number of bits. Also, the task-dedicated semantic information addresses prediction of caliper placement, so the trained encoder will try to find features in the cropped ultrasound image related to caliper location.

In block S619, the position embedding, the first image embedding, and the second image embedding are input to a trained second machine learning model. The trained second machine learning model is configured to predict positions of calipers in the cropped ultrasound image based on the preferred placements of the calipers in the matching template ultrasound image, as discussed above with reference to Fig. 5. In an embodiment, predicting the positions of the calipers in the cropped ultrasound image may include inputting the position embedding from block S616, the first image embedding from block S617, and the second image embedding from block S618 to the trained second machine learning model, and using the position embedding and the first image embedding from the matching template as prompts to guide the trained second machine learning model to predict the positions of the calipers in the cropped ultrasound image as represented by the second image embedding, which includes information about the input ultrasound image. That is, the trained second machine learning model applies self-attention and cross-attention to the position embedding, the first image embedding, and the second image embedding to determine relationships within and between the position embedding, the first image embedding, and the second image embedding for predicting the positions of the calipers in the cropped ultrasound image.

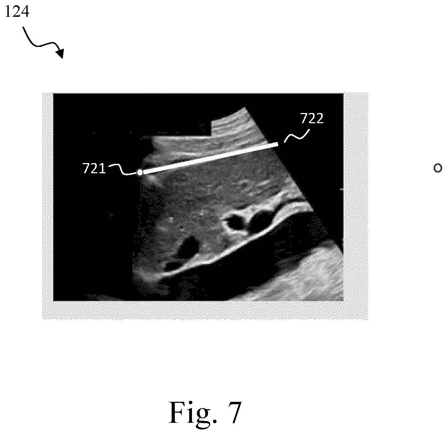

In block S620, the predicted positions of the calipers are displayed on a display. In an embodiment, the predicted positions of the calipers are displayed within the ultrasound image of the subject, which may be accomplished by translating or reprojecting coordinates of the predicted positions of the calipers in the cropped ultrasound image to corresponding coordinates in the original ultrasound image received in block S613, as would be apparent to one skilled in the art. This enables the user to accept or adjust the positions of the calipers in the original ultrasound image. Fig. 7 shows display 124 of the system 100, for example, displaying an illustrative original ultrasound image with the predicted positions of the calipers determined according to a representative embodiment. In particular, Fig. 7 depicts the predicted positions of first and second calibers 721 and 722 at key points in the displayed original ultrasound image of a liver, for example. The predicted positions of the first and second calibers 721 and 722 correspond to the user preference for performing a liver craniocaudal axis measurement determined during calibration. Alternatively, or in addition, the predicted positions of the calipers may be displayed within the cropped ultrasound image.

When accepted, the calipers may be placed at the predicted positions for measuring the anatomy of interest at the detected anatomical view using the ultrasound imaging system. When adjusted, the calipers may be placed at the adjusted positions for measuring the anatomy of interest. The predicted positions of the calipers may also be stored and/or output in a report, depending on the user's preferences.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although determining user-preferred positions of calipers in ultrasound images has been described with reference to exemplary embodiments, it is understood that the words that have been used are words of description and illustration, rather than words of limitation. Changes may be made within the purview of the appended claims, as presently stated and as amended, without departing from the scope and spirit of the embodiments. Also, although determining user-preferred positions of calipers in ultrasound images has been described with reference to particular means, materials and embodiments, it is not intended to be limited to the particulars disclosed; rather evaluating quality of an ultrasound imaging system extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A method of determining positions of calipers in ultrasound images, the method comprising:
performing calibration to identify preferred placements of calipers by a user in a plurality of template ultrasound images showing a plurality of anatomical views of anatomies, respectively (S611), wherein each anatomical view of the plurality of anatomical views corresponds to a type of measurement to be performed on an anatomy;
storing the preferred placements of the calipers in association with the plurality of template ultrasound images, respectively (S612);
receiving an ultrasound image of a subject acquired during an ultrasound examination (S613);
inputting the ultrasound image of the subject to a trained first machine learning model for automatically detecting an anatomical view of an anatomy of interest in the ultrasound image using object detection and cropping the ultrasound image to separately show a region of interest, ROI, that includes the detected anatomical view (S614), wherein the detected anatomical view corresponds to a type of measurement to be performed on the anatomy of interest;
retrieving a matching template ultrasound image of the plurality of template ultrasound images that matches the detected anatomical view of the anatomy of interest, and the preferred placements of the calipers stored in association with the matching template ultrasound image (S615);
encoding the preferred placements of the calipers to provide a position embedding representing positions of the preferred placements (S616);
encoding the matching template ultrasound image to provide a first image embedding representing the matching template ultrasound image (S617);
encoding the cropped ultrasound image separately showing the ROI to provide a second image embedding representing the cropped ultrasound image (S618);
inputting the position embedding, the first image embedding, and the second image embedding to a trained second machine learning model for predicting positions of calipers in the cropped ultrasound image based on the preferred placements of the calipers in the matching template ultrasound image (S619); and
displaying the predicted positions of the calipers in at least one of the ultrasound image or the cropped ultrasound image, enabling the user to accept or adjust the predicted positions of the calipers in the ultrasound image of the subject for measuring the anatomy of interest using the detected anatomical view (S620).

2. The method of claim 1, wherein performing the calibration to identify the preferred placements of the calipers, comprises:
displaying the plurality of template ultrasound images showing an anatomical view of the plurality of anatomical views; and
for each displayed template ultrasound image, receiving input from the user indicating positions of calipers for performing a measurement of the anatomy in the anatomical view shown in the displayed template ultrasound image, and storing the positions of the calipers as the preferred placements for performing the measurement of the anatomy.

3. The method of claim 1, wherein performing the calibration to identify the preferred placements of the calipers, comprises:
displaying the plurality of template ultrasound images showing an anatomical view of the plurality of anatomical views; and
for each displayed template ultrasound image, displaying a plurality of predetermined positions of calipers for performing a measurement of the anatomy in the anatomical view shown in the displayed template ultrasound image, receiving input from the user selecting one of the predetermined positions of the plurality of predetermined positions of the calipers for performing the measurement of the anatomy, and storing the selected one of the predetermined positions of the calipers as the preferred placements for performing the measurement of the anatomy.

4. The method of any of claims 1-3, wherein the trained first machine learning model is a You Only Look Once, YOLO, object detection model or a Single-Shot Multi-box Detection, SSD, model, and/or wherein the trained second machine learning model is a YOLO object detection model or an SSD model.

5. The method of any of claims 1-4, wherein encoding the preferred placements of the calipers to provide the position embedding comprises:
inputting coordinates of the preferred placements of the calipers to a mathematical model, which encodes the coordinates into a position multidimensional vector representing positions of the preferred placements.

6. The method of claim 5, wherein encoding the matching template ultrasound image to provide the first image embedding comprises:
inputting the matching template ultrasound image to a trained image encoding model, which encodes the matching template ultrasound image into a first image multidimensional vector representing at least first image semantic information of the matching template ultrasound image.

7. The method of claim 6, wherein encoding the cropped ultrasound image separately showing the ROI to provide the second image embedding comprises:
inputting the cropped ultrasound image to the trained image encoding model, which encodes the cropped ultrasound image into a second image multidimensional vector representing at least second image semantic information of the cropped ultrasound image.

8. The method of any of claims 1-7, wherein the trained second machine learning model is a transformer decoder model.

9. The method of any of claims 1-8, wherein predicting the positions of the calipers in the cropped ultrasound image comprises inputting the position embedding, the first image embedding, and the second image embedding, and using the position embedding and the first image embedding as prompts to guide the trained second machine learning model to predict the positions of the calipers in the cropped ultrasound image.

10. The method of claim 9, wherein the trained second machine learning model applies self-attention and cross-attention to the position embedding, the first image embedding, and the second image embedding to determine relationships within and between the position embedding, the first image embedding, and the second image embedding for predicting the positions of the calipers in the cropped ultrasound image.

11. A system for determining positions of calipers in ultrasound images, the system comprising:
a display (124);
a processing unit (120) coupled to the display; and
a non-transitory memory (130) storing instructions that, when executed by the processing unit, cause the processing unit to perform the steps of a method of any of claims 1-10.

12. The system of claim 11, wherein the display is adapted to display the plurality of template ultrasound images.

13. The system of claim 12, wherein the display is further adapted to display, for each displayed template ultrasound image, a plurality of predetermined positions of calipers for performing a measurement of the anatomy in the detected anatomical view shown in the displayed template ultrasound image.

14. A non-transitory computer readable medium storing instructions for determining positions of calipers in ultrasound images that, when executed by a processing unit, cause the processing unit to perform the steps of a method of any of claims 1-10.
